# EUROPÄISCHE PATENTSCHRIFT

(11) **EP 0 659 748 B1**
(45) Veröffentlichungstag und Bekanntmachung des Hinweises auf die Patenterteilung: **12.07.2000**
(21) Anmeldenummer: 94120069.3
(22) Anmeldetag: 19.12.1994
(51) Int. Cl.: C07D 279/02, C07D 217/24, C07C 311/16, C07C 233/65, A61K 31/54, A61K 31/47

(54) **Substituierte 1,1-dioxo-2H-1,2-benzothiazinoylguanidine mit Na+/H+-Austausch inhibierender Wirkung**
Substituted 1,1,-dioxo-2H-1,2-benzothiazinoylguanidines with Na+/H+-exchange inhibiting activity
1,1-dioxo-2H-1,2-benzothiazinoylguanidines substituées ayant une activité d'inhibition l'échange Na+ /H+

(30) Priorität: 24.12.1993 DE 4344550
(43) Veröffentlichungstag der Anmeldung: 28.06.1995
(73) Patentinhaber: HOECHST AKTIENGESELLSCHAFT, 65929 Frankfurt am Main (DE)
(72) Erfinder: Weichert, Andreas, Dr., D-63329 Egelsbach (DE); Kleemann, Heinz-Werner, Dr., D-65474 Bischofsheim (DE); Lang, Hans-Jochen, Dr., D-65719 Hofheim (DE); Schwark, Jan-Robert, Dr., D-65929 Frankfurt (DE); Albus, Udo, Dr., D-61197 Florstadt (DE); Scholz, Wolfgang, Dr., D-65760 Eschborn (DE)

(56) Entgegenhaltungen:
- EP-A- 0 416 499
- EP-A- 0 556 674
- EP-A- 0 589 336
- EP-A- 0 602 523

## Beschreibung

Die Erfindung betrifft Acylguanidine der Formel I 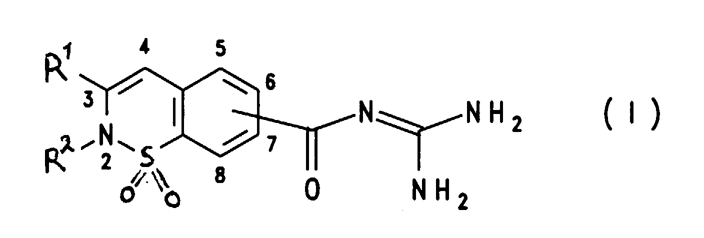 worin bedeuten:
- R¹: (C₃-C₈)-Cycloalkyl oder Phenyl,
das unsubstituiert oder substituiert ist mit 1 - 3 Substituenten aus der Gruppe F, Cl, CF₃, CH₃, Methoxy, Hydroxy, Amino, Methylamino und Dimethylamino;
- R²: H oder (C₁-C₄)-Alkyl;
sowie deren pharmazeutisch verträgliche Salze.

Besonders bevorzugt sind Verbindungen der Formel I, in denen das Carbonylguanidid [-CO-N = C(NH₂)₂] in 7-Stellung am jeweiligen Benzolkern situiert ist.

Enthält einer der Substituenten R¹ und R² ein oder mehrere Asymmetriezentren, so können diese sowohl S als auch R konfiguriert sein. Die Verbindungen können als optische Isomere, als Diastereomere, als Racemate oder als Gemische derselben vorliegen.

Die bezeichneten Alkylreste können sowohl geradkettig als auch verzweigt vorliegen.

Die Erfindung betrifft weiterhin ein Verfahreh zur Herstellung der Verbindung I, dadurch gekennzeichnet, daß man
Verbindungen der Formel II 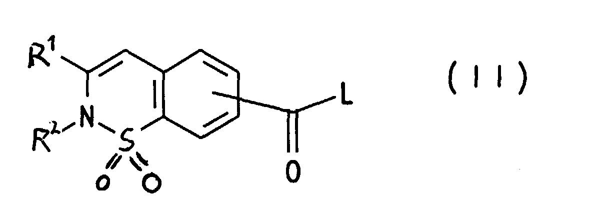 worin R¹ und R² die angegebene Bedeutung besitzen und L für eine leicht nucleophil substituierbare Fluchtgruppe steht,
die ausgewählt ist aus der Gruppe bestehend aus Methoxy, Phenoxy, Phenylthio, Methylthio, 2-Pyridylthio, 1-Imidazolyl, -OCOOEthyl, -O-SO₂-p-Tolyl und -O-C( = N-Cyclohexyl)-(NH-Cyclohexyl)
mit Guanidin umsetzt.

Die Erfindung betrifft außerdem Verbindungen der Formel II 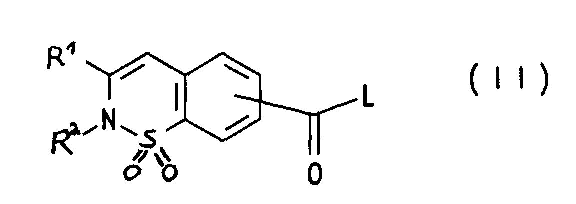 worin R¹ und R² die angegebene Bedeutung besitzen und L für eine leicht nucleophil substituierbare Fluchtgruppe steht,
die ausgewählt ist aus der Gruppe bestehend aus Methoxy, Phenoxy, Phenylthio, Methylthio, 2-Pyridylthio, 1-Imidazolyl, -OCOOEthyl, -O-SO₂-p-Tolyl und -O-C( = N-Cyclohexyl)-(NH-Cyclohexyl).

Die aktivierten Säurederivate der Formel II, worin L eine Alkoxy-, vorzugsweise eine Methoxygruppe, eine Phenoxygruppe, Phenylthio-, Methylthio-, 2-Pyridylthiogruppe, einen Stickstoffheterocyclus, vorzugsweise 1-Imidazolyl, bedeutet, erhält man vorteilhaft in an sich bekannter Weise aus den zugrundeliegenden Carbonsäurechloriden (Formel II, L = Cl), die man ihrerseits wiederum in an sich bekannter Weise aus den zugrundeliegenden Carbonsäuren (Formel II, L = OH) beispielsweise mit Thionylchlorid herstellen kann.

Neben den Carbonsäurechloriden der Formel II (L = Cl) lassen sich auch weitere aktivierte Säurederivate der Formel II in an sich bekannter Weise direkt aus den zugrundeliegenden Carbonsäure-derivaten (Formel II, L = OH) herstellen, wie beispielsweise die Methylester der Formel II mit L = OCH₃ durch Behandeln mit gasförmigem HCl in Methanol, die Imidazolide der Formel II durch Behandeln mit Carbonyldiimidazol [L = 1-Imidazolyl, Staab, Angew. Chem. Int. Ed. Engl. 1, 351 -367 (1962)], die gemischten Anhydride II mit Cl-COOC₂H₅ oder Tosylchlorid in Gegenwart von Triethylamin in einem inerten Lösungsmittel, wie auch die Aktivierungen von Carbonsäuren mit Dicyclohexylcarbodiimid (DCC) oder mit O-[(Cyano(ethoxycarbonyl)methylen)amino]-1,1,3,3-tetramethyluronium-tetrafluor borat ("TOTU"), [Proceedings of the 21. European Peptide Symposium, Peptides 1990, Editors E. Giralt and D. Andreu, Escom, Leiden, 1991]. Eine Reihe geeigneter Methoden zur Herstellung von aktivierten Carbonsäurederivaten der Formel II sind unter Angabe von Quellenliteratur in J. March, Advanced Organic Chemistry, Third Edition (John Wiley & Sons, 1985), S. 350 angegeben.

Die Umsetzung eines aktivierten Carbonsäurederivates der Formel II mit Guanidin erfolgt in an sich bekannter Weise in einem protischen oder aprotischen polaren aber inerten organischen Lösungsmittel. Dabei haben sich bei der Umsetzung der Carbonsäuremethylester (II, L = OMe) mit Guanidin Methanol, Isopropanol oder THF von 20°C bis zur Siedetemperatur dieser Lösungsmittel bewährt. Bei den meisten Umsetzungen von Verbindungen II mit salzfreiem Guanidin wurde vorteilhaft in aprotischen inerten Lösungsmitteln wie THF, Dimethoxyethan, Dioxan gearbeitet. Aber auch Wasser kann unter Gebrauch einer Base wie beispielsweise NaOH als Lösungsmittel bei der Umsetzung von II mit Guanidin verwendet werden.

Wenn L = Cl bedeutet, arbeitet man vorteilhaft unter Zusatz eines Säurefängers, z. B. in Form von überschüssigen Guanidin zur Abbindung der Halogenwasserstoffsäure.

Die zugrundeliegenden Carbonsäurederivate der Formel II sind unbekannt und werden wie folgt dargestellt:

### Derivate der Formel II:

Chlorsulfonierung der jeweiligen stellungsisomeren Brom- bzw. Iod-Carbonsäuren in reiner Chlor-sulfonsäure bei 80° - 120°C über 3 - 8 h ergibt die entsprechenden Brom-bzw. Iod-substituierten Sulfo-chloride. Um N-alkylsubstituierte Sulfonamide [Formel IV, R(2) ungleich H] herzustellen, wird das jeweilige Sulfochlorid mit einem Überschuß an primärem Amin bzw. an primärem Amin-hydro-chlorid in 1 ON NaOH (aq) bei Raumtemperatur innerhalb 3 - 6 h umgesetzt. Saure, wäßrige Aufarbeitung liefert die substituierten Sulfonamide. Im Fall von unsubstituierten Sulfonamiden (Formel IV, R(2) gleich H) arbeitet man in flüssigem Ammoniak. Zu Verbindungen der Formel IV gelangt man schließlich, indem die Carbonsäurefunktionalität in bekannter Weise in eine Gruppe -CO-L überführt wird, wobei L vorzugsweise eine Methoxygruppe darstellt. In an sich bekannter Weise gelingt die Darstellung der Verbindungen der Formel III durch die Einführung der Acetylenfunktionalität. Dabei werden flüssige, substituierte Acetylene [R(1) ungleich H] in THF bei Raumtemperatur in Gegenwart von n-Butylamin, Kupfer(I)iodid und katalytischen Mengen an Bis(triphenylphosphin)-palladium(II)-chlorid mit Verbindungen der Formel IV gekoppelt. Im Fall von Acetylen [R(1) gleich H] wird dieses für 3 - 5 h durch die Reaktionsmischung geleitet. Die Verbindungen der Formel II werden nun durch die erfindungsgemäße, bei Raumtemperatur durchzuführende Quecksilber(II)acetat (0.3eq) vermittelte Cyclisierung in Eisessig in Gegenwart von Schwefelsäure als Katalysator erhalten.

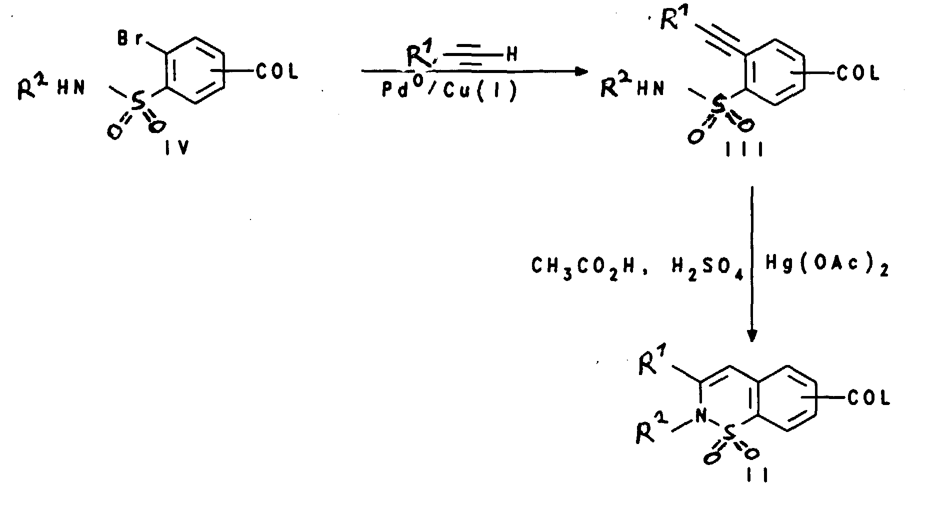

Die erhaltenen Carbonsäurederivate werden nach einer der oben beschriebenen Verfahrensvarianten zu erfindungsgemäßen Verbindungen I umgesetzt.

Acylguanidine I sind im allgemeinen schwache Basen und können Säure unter Bildung von Salzen binden. Als Säureadditionssalze kommen Salze aller pharmakologisch verträglichen Säuren infrage, beispielsweise Halogenide, insbesondere Hydrochloride, Lactate, Sulfate, Citrate, Tartrate, Acetate, Phosphate, Methylsulfonate, p-Toluolsulfonate.

Die Verbindungen I sind substituierte Acylguanidine.

Prominentester Vertreter der Acylguanidine ist das Pyrazinderivat Amilorid, das als kaliumsparendes Diuretikum in der Therapie Verwendung findet. Zahlreiche weitere Verbindungen vom Amilorid-Typ werden in der Literatur beschrieben, wie beispielsweise Dimethylamilorid oder Ethylisopropylamilorid. 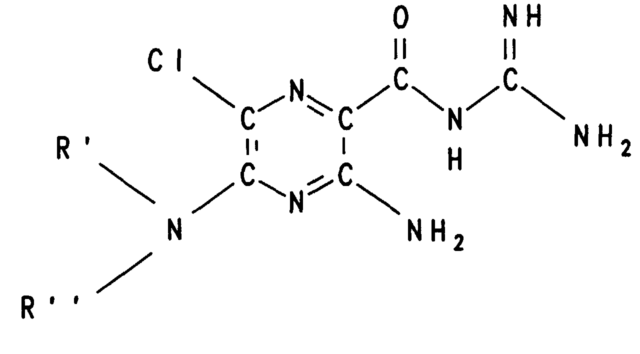
Amilorid: R',R'' = H
Dimethylamilorid: R',R'' = CH₃
Ethylisopropylamilorid: R' = C₂H₅, R'' = CH(CH₃)₂

Darüberhinaus sind Untersuchungen bekannt geworden, die auf antiarrhythmische Eigenschaften von Amilorid hinweisen (Circulation 79, 1257 - 63 (1989)). Einer breiten Anwendung als Antiarrhythmikum steht jedoch entgegen, daß dieser Effekt nur schwach ausgeprägt ist und von einer blutdrucksenkenden und saluretischen Wirkung begleitet auftritt und diese Nebenwirkungen bei der Behandlung von Herz-Rhythmusstörungen unerwünscht sind.

Hinweise auf antiarrhythmische Eigenschaften des Amilorids wurden auch bei Experimenten an isolierten Tierherzen erhalten (Eur. Heart J. 9 (suppl.1): 167 (1988), book of abstracts). So wurde beispielsweise an Rattenherzen gefunden, daß ein künstlich ausgelöstes Kammerflimmern durch Amilorid völlig unterdrückt werden konnte. Noch potenter als Amilorid war in diesem Modell das oben erwähnte Amiloridderivat Ethylisopropylamilorid.

In der US-Patentschrift 5 091 394 (EP 416 499; HOE 89/F 288) und in der europäischen Offenlegungsschrift 0 556 674 (HOE 92/F 034) werden Benzoylguanidine beschrieben, jedoch wird darin kein Hinweis auf bicyclische heterocyclische Verbindungen gegeben.

Im US-Patent 3 780 027 werden Acylguanidine beansprucht, die sich von im Handel befindlichen Schleifendiuretika, wie Bumetanid, ableiten. Entsprechend wird für diese Verbindungen eine starke salidiuretische Wirksamkeit berichtet. EP-A-0 589 336 und EP-A-0 602 523 sind Stand der Technik im sinne von Artikel 54(3) EPÜ.

Es war daher überraschend, daß die erfindungsgemäßen Verbindungen keine unerwünschten und nachteiligen salidiuretischen, jedoch sehr gute antiarrhythmische Eigenschaften aufweisen, besonders in Situationen, wie sie beispielsweise bei Sauerstoffmangelerscheinungen auftreten. Die Verbindungen sind infolge ihrer pharmakologischen Eigenschaften als antiarrhythmische Arzneimittel mit cardioprotektiver Komponente zur Infarktprophylaxe und der Infarktbehandlung sowie zur Behandlung der angina pectoris hervorragend geeignet, wobei sie auch präventiv die pathophysiologischen Vorgänge beim Entstehen ischämisch induzierter Schäden, insbesondere bei der Auslösung ischämisch induzierter Herzarrhythmien, inhibieren oder stark vermindern. Wegen ihrer schützenden Wirkungen gegen pathologische hypoxische und ischämische Situationen können die erfindungsgemäßen Verbindungen der Formel I infolge Inhibition des zellulären Na⁺ /H⁺ -Austauschmechanismus als Arzneimittel zur Behandlung aller akuten oder chronischen durch Ischämie ausgelösten Schäden oder dadurch primär oder sekundär induzierten Krankheiten verwendet werden. Dies betrifft ihre Verwendung als Arzneimittel für operative Eingriffe, z.B. bei Organ-Transplantationen, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den Empfängerorganismus verwendet werden können. Die Verbindungen sind ebenfalls wertvolle, protektiv wirkende Arzneimittel bei der Durchführung angioplastischer operativer Eingriffe beispielsweise am Herzen wie auch an peripheren Gefäßen. Entsprechend ihrer protektiven Wirkung gegen ischämisch induzierte Schäden sind die Verbindungen auch als Arzneimittel zur Behandlung von Ischämien des Nervensystems, insbesondere des ZNS, geeignet, wobei sie z.B. zur Behandlung des Schlaganfalls oder des Hirnödems geeignet sind. Darüberhinaus eignen sich die erfindungsgemäßen Verbindungen der Formel I ebenfalls zur Behandlungen von Formen des Schocks, wie beispielweise des allergischen, cardiogenen, hypovolämischen und des bakteriellen Schocks.

Darüberhinaus zeichnen sich die erfindungsgemäßen Verbindungen der Formel I durch starke inhibierende Wirkung auf die Proliferationen von Zellen, beispielsweise der Fibroblasten- Zeliproliferation und der Proliferation der glatten Gefäßmuskelzellen, aus. Deshalb kommen die Verbindungen der Formel I als wertvolle Therapeutika für Krankheiten infrage, bei denen die Zellproliferation eine primäre oder sekundäre Ursache darstellt, und können deshalb als Antiatherosklerotika, Mittel gegen diabetische Spätkomplikationen, Krebserkrankungen, fibrotische Erkrankungen wie Lungenfibrose, Leberfibrose oder Nierenfibrose, Organhypertrophien und -hyperplasien, insbesondere bei Prostatahyperplasie bzw. Prostatahypertrophie verwendet werden.

Die erfindungsgemäßen Verbindungen sind wirkungsvolle Inhibitoren des zellulären Natrium-Protonen-Antiporters (Na⁺/H⁺ -Exchanger), der bei zahlreichen Erkrankungen (Essentielle Hypertonie, Atherosklerose, Diabetes usw.) auch in solchen Zellen erhöht ist, die Messungen leicht zugänglich sind, wie beispielsweise in Erythrocyten, Thrombocyten oder Leukozyten. Die erfindungsgemäßen Verbindungen eignen sich deshalb als hervorragende und einfache wissenschaftliche Werkzeuge, beispielsweise in ihrer Verwendung als Diagnostika zur Bestimmung und Unterscheidung bestimmter Formen der Hypertonie, aber auch der Atherosklerose, des Diabetes, proliferativer Erkrankungen usw.. Darüber hinaus sind die Verbindungen der Formel I für die präventive Therapie zur Verhinderung der Genese des Bluthochdrucks, beispielweise der essentiellen Hypertonie, geeignet.

Arzneimittel, die eine Verbindung I enthalten, können dabei oral, parenteral, intravenös, rektal oder durch Inhalation appliziert werden, wobei die bevorzugte Applikation von dem jeweiligen Erscheinungsbild der Erkrankung abhängig ist. Die Verbindungen I können dabei allein oder zusammen mit galenischen Hilfsstoffen zur Anwendung kommen, und zwar sowohl in der Veterinär- als auch in der Humanmedizin.

Welche Hilfsstoffe für die gewünschte Arzneimittelformulierung geeignet sind, ist dem Fachmann auf Grund seines Fachwissens geläufig. Neben Lösemitteln, Gelbildnern, Suppositorien-Grundlagen, Tablettenhilfastoffen, und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wäßrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen oder intravenösen Applikation werden die aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z. B. in Frage: Wasser, physiologische Kochsalzlösung oder Alkohole, z. B. Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierung für die Verabreichung in Form von Aerosolen oder Sprays sind geeignet z. B. Lösungen, Suspensionen oder Emulsionen des Wirkstoffes der Formel I in einem pharmazeutisch unbedenklichen Lösungsmittels, wie insbesondere Ethanol oder Wasser, oder einem Gemisch solcher Lösungsmittel.

Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.

Die Dosierung des zu verabreichenden Wirkstoffs der Formel I und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers.
Im Durchschnitt beträgt die tägliche Dosis einer Verbindung der Formel I bei einem etwa 75 kg schweren Patienten mindestens 0,001 mg/kg, vorzugsweise 0,01 mg/kg, bis höchstens 10 mg/kg, vorzugsweise 1 mg/kg Körpergewicht. Bei akuten Ausbrüchen der Krankheit, etwa unmittelbar nach Erleiden eines Herzinfarkts, können auch noch höhere und vor allem häufigere Dosierungen notwendig sein, z. B. bis zu 4 Einzeldosen pro Tag. Insbesondere bei i.v. Anwendung, etwa bei einem Infarktpatienten auf der Intensivatation können bis zu 200 mg pro Tag notwendig werden.

Liste der Abkürzungen:
- MeOH: Methanol
- DMF: N,N-Dimethylformamid
- RT: Raumtemperatur
- EE: Ethylacetat (EtOAc)
- Smp: Schmelzpunkt
- THF: Tetrahydrofuran
- eq.: Äquivalent

### Experimenteller Teil

### Allgemeine Vorschrift zur Herstellung von Aroyl-guanidinen (I)

### Variante A: aus Carbonsäuren (II, L = OH)

1.0 eq. des Carbonsäurederivates der Formel II löst bzw. suspendiert man in wasserfreiem THF (5 ml/mmol) und versetzt sodann mit 1.1 eq. Carbonyldiimidazol. Nach dem Rühren über 2 Stunden bei RT werden 5.0 eq. Guanidin in die Reaktionslösung eingetragen. Nach dem Rühren über Nacht destilliert man das THF unter vermindertem Druck (Rotavapor) ab, versetzt mit Wasser, stellt mit 2 N HCl auf pH 6 bis 7 und filtriert das entsprechende Acylguanidin (Formel I) ab. Die so erhaltenen Acylguanidine können durch Behandeln mit wäßriger, methanolischer oder etherischer Salzsäure oder anderen pharmakologisch verträglichen Säuren in die entsprechenden Salze übergeführt werden.

### Allgemeine Vorschrift zur Herstellung von Aroyl-guanidinen (I)

### Variante B: aus Carbonsäure-alkylestern (II, L = O-Alkyl)

1.0 eq. des Carbonsäure-alkylesters der Formel II sowie 5.0 eq. Guanidin (freie Base) werden in Isopropanol gelöst oder in THF suspendiert und bis zum vollständigen Umsatz (Dünnschichtkontrolle) zum Rückfluß erhitzt (typische Reaktionszeit 2 bis 5 h). Das Lösungsmittel wird unter vermindertem Druck (Rotavapor) abdestilliert in EE aufgenommen und 3 x mit NaHCO₃-Lösung gewaschen. Es wird über Na₂SO₄ getrocknet, das Lösungsmittel im Vakuum abdestilliert und an Kieselgel mit einem geeigneten Laufmittel, z. B. EE/MeOH 5 : 1 chromatographiert.
(Salzbildung vergleiche Variante A)

### Beispiel 1:

### 1,1-Dioxo-3-phenyl-2H-1,2-benzothiazin-7-carbonsäureguanidid-hydrochlorid Farblose Kristalle, Smp. 271-73°C

### Syntheseweg:

a) 4-Brom-3-chlorsulfonyl-benzoesäure aus 4-Brom-benzoesäure durch Erhitzen auf 120°C in reiner Chlorsulfonsäure für 9 h. Gießen auf Eis ergibt farblose Kristalle, Smp. 185 - 190°C.
b) 4-Brom-3-sulfamoyl-benzoesäure aus a) durch Aminolyse in flüssigem Ammoniak bei -40°C und anschließendes Erwärmen auf RT. Wäßrige Aufarbeitung gibt farblose Kristalle, Smp. > 250°C
c) 4-Brom-3-sulfamoyl-benzoesäuremethylester aus b) durch Veresterung in Methanol in Gegenwart von Acetylchlorid, farblose Kristalle, Smp. 151°C
d) 4-Phenylalkinyl-3-sulfamoyl-benzoesäuremethylester aus c) durch Stephans-Castro-coupling mit 2.5 Äquivalenten Phenylacetylen , Rühren bei RT für 24 h in Gegenwart von katal. (5 mol%) Bis-(Triphenylphosphin)-palladium(II)chlorid, 15 mol% Kupfer(I)iodid und 3 Äquivalenten n-Butylamin, Aufarbeitung mit wäßrigem Ammoniumchlorid, Extraktion mit Essigester und anschließende Säulenchromatographie an Kieselgel mit Essigester/n-Heptan, farblose Kristalle, Smp. 142°C.
e) 1,1-Dioxo-3-phenyl-2H-1,2-benzothiazin-7-carbonsäuremethylester aus d) durch Hg(OAc)₂-vermittelte Cyclisierung in Eisessig in Gegenwart von konz. Schwefelsäure bei Zimmertemperatur für 3 Stunden. Absaugen und Verreiben in Ether ergibt farblose Kristalle, Smp. 240°C
f) 1,1-Dioxo-3-phenyl-2H-1,2-benzothiazin-7-carbonsäureguanidid-hydrochlorid aus e) nach allgemeiner Vorschrift B

### Beispiel 2:

### 3-Cyclohexyl-1,1-dioxo-2H-1,2-benzothiazin-7-carbonsäureguanidid-hydrochlorid Farblose Kristalle, Smp. 265 - 67°C

### Syntheseweg:

a) 4-Cyclohexylalkinyl-3-sulfamoyl-benzoesäuremethylester aus 1 c) analog Verfahren 1 d), jedoch unter Verwendung von Cyclohexylacetylen, farblose Kristalle, Smp. 115 - 18°C.
b) 3-Cyclohexyl-1,1-dioxo-2H-1,2-benzothiazin-7-carbonsäuremethylester aus a) analog Verfahren 1 d), farblose Kristalle, Smp. 170 - 72°C
c) 3-Cyclohexyl-1,1-dioxo-2H-1,2-benzothiazin-7-carbonsäureguanidid-hydrochlorid aus b)
   nach allgemeiner Vorschrift B

### Beispiel 3

### 1,1-Dioxo-2-methyl-3-phenyl-2H-1,2-benzothiazin-7-oyl-guanidin-hydrochlorid Farblose Kristalle, Smp. 243 - 45°C

### Syntheseweg:

a) 4-Brom-3-(N-methyl-sulfamoyl)-benzoesäuremethylester
   aus 4-Brom-3-chlorsulfonylbenzoesäuremethylester analog Verfahren 1 b), jedoch unter Einsatz von Methylamin,
   farblose Kristalle, Smp. 167 - 69°C.
b) 4-(Phenyl-ethinyl)-3-(N-methyl-sulfamoyl)-benzoesäuremethylester
   aus a) analog Verfahren 1 d),
   farblose Kristalle, Smp. 165 - 67°C.
c) 1,1-Dioxo-2-methyl-3-phenyl-2H-1,2-benzothiazin-7-carbonsäuremethylester
   aus a) analog Verfahren 1 e),
   farblose Kristalle, Smp. 145 - 47°C
d) 1,1-Dioxo-2-methyl-3-phenyl-2H-1,2-benzothiazin-7-oyl-guanidin-hydrochlorid
   aus c) nach allgemeiner Vorschift B

### Pharmakologische Daten.

### Inhibition des Na⁺ /H⁺ -Exchangers von Kaninchenerythrocyten

Weiße Neuseeland-Kaninchen (Ivanovas) erhielten eine Standard-Diät mit 2% Cholesterin für sechs Wochen, um den Na⁺ /H⁺ -Austausch zu aktivieren und so den Na⁺ -Influx in die Erythrocyten via Na⁺ /H⁺ -Austausch flammenphotometrisch bestimmen zu können. Das Blut wurde den Ohrarterien entnommen und durch 25 IE Kalium-Heparin ungerinnbar gemacht. Ein Teil jeder Probe wurde zur Doppelbestimmung des Hämatokrits durch Zentrifugieren benutzt. Aliquots von jeweils 100 µl dienten zur Messung des Na⁺ -Ausgangsgehalts der Erythrocyten.

Um den Amilorid-sensitiven Natrium-Influx zu bestimmen, wurden 100 µl jeder Blutprobe in jeweils 5 ml eines hyperosmolaren Salz-Sucrose-Mediums (mmol/l: 140 NaCl, 3 KCl, 150 Sucrose, 0,1 Ouabain, 20 Tris-hydroxymethyl-aminomethan) bei pH 7,4 und 37°C inkubiert. Die Erythrocyten wurden danach dreimal mit eiskalter MgCl₂-Ouabain-Lösung (mmol/l: 112 MgCl₂, 0,1 Ouabain) gewaschen und in 2,0 ml destilliertem Wasser hämolysiert. Der intrazelluläre Natriumgehalt wurde flammenphotometrisch bestimmt.

Der Na⁺ -Nettoinflux wurde aus der Differenz zwischen Natrium-Ausgangswerten und dem Natriumgehalt der Erythrocyten nach Inkubation errechnet. Der Amiloridhemmbare Natrium-Influx ergab sich aus der Differenz des Natriumgehalts der Erythrocyten nach Inkubation mit und ohne Amilorid 3 x 10⁻⁴ mol/l. Auf diese Weise wurde auch bei den erfindungsgemäßen Verbindungen verfahren.

### Ergebnisse

### Inhibition des Na⁺ /H⁺ -Exchangers:

- Beispiel 1:: IC₅₀ = 0.9 x 10⁻⁶ mol /l
- Beispiel 2:: IC₅₀ = 0.5 x 10⁻⁶ mol/l

## Patentansprüche

1. Acylguanidine der Formel I 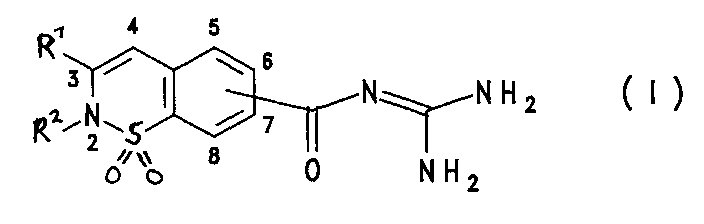 worin bedeuten:
R¹ (C₃-C₈)-Cycloalkyl oder Phenyl,
das unsubstituiert oder substituiert ist mit 1 - 3 Substituenten aus der Gruppe F, Cl, CF₃, CH₃, Methoxy, Hydroxy, Amino, Methylamino, Dimethylamino;
R² H oder (C₁-C₄)-Alkyl;
sowie deren pharmazeutisch verträgliche Salze

2. Verbindungen der Formel I nach Anspruch 1, in denen das Carbonylguanidid [-CO-N=C(NH₂)₂] in 7-Stellung am jeweiligen Benzolkern situiert ist.

3. Verfahren zur Herstellung einer Verbindung I nach Anspruch 1, dadurch gekennzeichnet, daß man eine Verbindung der Formel II 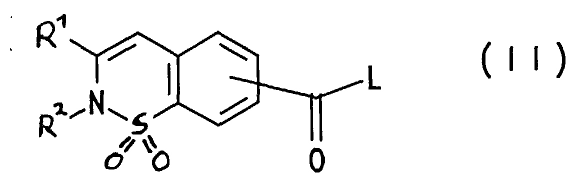 worin R¹ und R² die in Anspruch 1 angegebene Bedeutung besitzen und L für eine leicht nucleophil substituierbare Fluchtgruppe steht,
die ausgewählt ist aus der Gruppe bestehend aus Methoxy, Phenoxy, Phenylthio, Methylthio, 2-Pyridylthio, 1-Imidazolyl, -OCOOEthyl, -O-SO₂-p-Tolyl und -O-C(=N-Cyclohexyl)-(NH-Cyclohexyl) mit Guanidin umsetzt.

4. Verbindung der Formel II 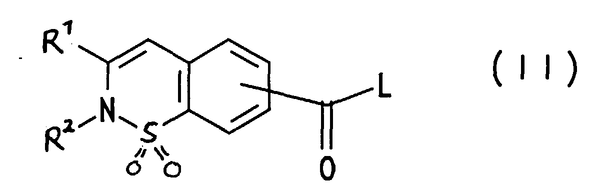 worin R¹ und R² die in Anspruch 1 angegebene Bedeutung besitzen und
L Methoxy, Phenoxy, Phenylthio, Methylthio, 2-Pyridylthio, 1-Imidazolyl, -OCOOEthyl, -O-SO₂-p-Tolyl oder -O-C(=N-Cyclohexyl)-(NH-Cyclohexyl);

5. Verwendung einer Verbindung I nach Anspruch 1 zur Herstellung eines Medikaments zu Behandlung von Arrhythmien.

6. Verwendung einer Verbindung I nach Anspruch 1 zur Herstellung eines Medikaments zur Behandlung oder Prophylaxe des Herzinfarktes.

7. Verwendung einer Verbindung I nach Anspruch 1 zur Herstellung eines Medikaments zur Behandlung oder Prophylaxe der Angina Pectoris.

8. Verwendung einer Verbindung I nach Anspruch 1 zur Herstellung eines Medikaments zur Behandlung oder Prophylaxe von ischämischen Zuständen des Herzens.

9. Verwendung einer Verbindung I nach Anspruch 1 zur Herstellung eines Medikaments zur Behandlung oder Prophylaxe von ischämischen Zuständen des peripheren und zentralen Nervensystems und des Schlaganfalls.

10. Verwendung einer Verbindung I nach Anspruch 1 zur Herstellung eines Medikaments zur Behandlung oder Prophylaxe von ischämischen Zuständen peripherer Organe und Gliedmaßen.

11. Verwendung einer Verbindung I nach Anspruch 1 zur Herstellung eines Medikaments zur Behandlung von Schockzuständen.

12. Verwendung einer Verbindung I nach Anspruch 1 zur Herstellung eines Medikaments zum Einsatz bei chirurgischen Operationen und Organtransplantationen.

13. Verwendung einer Verbindung I nach Anspruch 1 zur Herstellung eines Medikaments zur Konservierung und Lagerung von Transplantaten für chirurgische Maßnahmen.

14. Verwendung einer Verbindung I nach Anspruch 1 zur Herstellung eines Medikaments zur Behandlung von Krankheiten, bei denen die Zellproliferation eine primäre oder sekundäre Ursache darstellt.

15. Heilmittel, welches eine wirksame Menge einer Verbindung der Formel I nach Anspruch 1 und übliche Zusatzstoffe enthält.

## Claims

1. An acylguanidine of the formula I 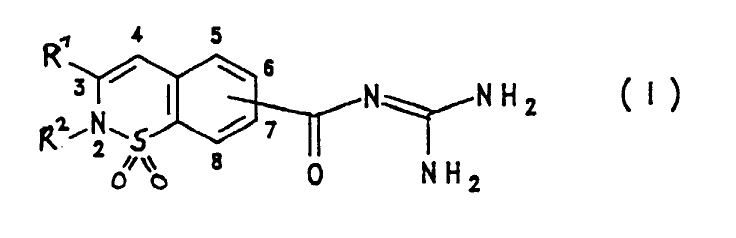 in which:
R₁ is (C₃-C₈)-cycloalkyl or phenyl,
which is unsubstituted or substituted by 1-3 substituents from the group consisting of F, Cl, CF₃, CH₃, methoxy, hydroxyl, amino, methylamino, dimethylamino;
R₂ is H or (C₁-C₄)-alkyl;
and the pharmaceutically suitable salts thereof.

2. A compound of the formula I as claimed in claim 1, in which the carbonyl guanidide [-CO-N=C(NH₂)₂] is situated in position 7 on the particular benzene nucleus.

3. A process for the preparation of a compound I as claimed in claim 1, which comprises reacting a compound of the formula II 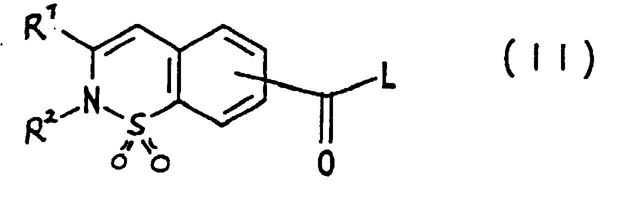 in which R₁ and R₂ have the meaning stated in claim 1 and L is a leaving group which can easily undergo nucleophilic substitution and which is selected from the group consisting of methoxy, phenoxy, phenylthio, methylthio, 2-pyridylthio, 1-imidazolyl, -OCOOethyl, -O-SO₂-p-tolyl and -O-C(=N-cyclohexyl) - (NH-cyclohexyl) with guanidine.

4. A compound of the formula II 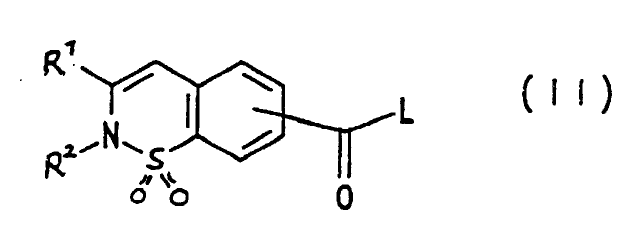 in which R₁ and R₂ have the meaning stated in claim 1 and
L is methoxy, phenoxy, phenylthio, methylthio, 2- pyridylthio, 1-imidazolyl, -OCOOethyl, -O-SO₂-p-tolyl or -O-C(=N-cyclohexyl)-(NH-cyclohexyl).

5. The use of a compound I as claimed in claim 1 for the production of a medicament for the treatment of arrhythmias.

6. The use of a compound I as claimed in claim 1 for the production of a medicament for the treatment or prophylaxis of myocardial infarct.

7. The use of a compound I as claimed in claim 1 for the production of a medicament for the treatment or prophylaxis of angina pectoris.

8. The use of a compound I as claimed in claim 1 for the production of a medicament for the treatment or prophylaxis of ischemic states of the heart.

9. The use of a compound I as claimed in claim 1 for the production of a medicament for the treatment or prophylaxis of ischemic states of the peripheral and central nervous system and of stroke.

10. The use of a compound I as claimed in claim 1 for the production of a medicament for the treatment or prophylaxis of ischemic states of the peripheral organs and limbs.

11. The use of a compound I as claimed in claim 1 for the production of a medicament for the treatment of states of shock.

12. The use of a compound I as claimed in claim 1 for the production of a medicament for use in surgical operations and organ transplants.

13. The use of a compound I as claimed in claim 1 for the production of a medicament for the preservation and storage of transplants for surgical procedures.

14. The use of a compound I as claimed in claim 1 for the production of a medicament for the treatment of diseases in which cell proliferation represents a primary or secondary cause.

15. A medicine which comprises an effective amount of a compound of the formula I as claimed in claim 1 and conventional additives.

## Revendications

1. Acylguanidines de formule I 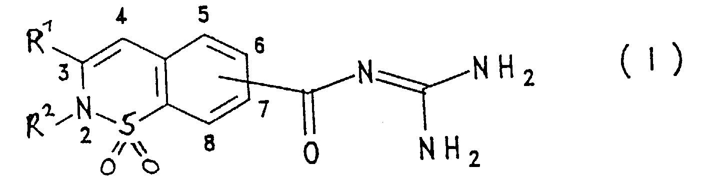 dans laquelle
R¹ représente un groupe cycloalkyle en C₃ -C₈ ou phényle,
qui n'est pas substitué ou porte 1 à 3 substituants choisis dans l'ensemble constitué par F, Cl, CF₃, CH₃ et les groupes méthoxy, hydroxyle, amino, méthylamino, diméthylamino;
R² représente H ou un groupe alkyle en C₁ -C₄;
et leurs sels pharmaceutiquement acceptables.

2. Composés de formule I selon la revendication 1, dans lesquels le fragment carbonylguanidide [-CO-N=C(NH₂)₂] se trouve en position 7 sur le cycle benzénique respectif.

3. Procédé pour la préparation d'un composé I selon la revendication 1, caractérisé en ce que l'on fait réagir avec de la guanidine un composé de formule II 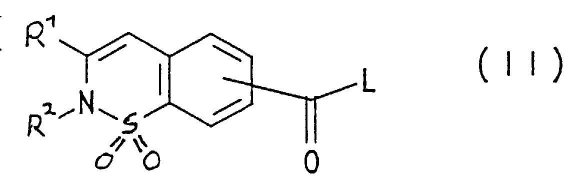 dans laquelle R¹ et R² ont les significations données dans la revendication 1 et L représente un groupe partant pouvant être aisément remplacé par substitution nucléophile,
qui est choisi dans l'ensemble constitué par les groupes méthoxy, phénoxy, phénylthio, méthylthio, 2-pyridylthio, 1-imidazolyle, -OCOO-éthyle, -O-SO₂-p-tolyle et -O-C(=N-cyclohexyl)-(NH-cyclohexyle).

4. Composé de formule II 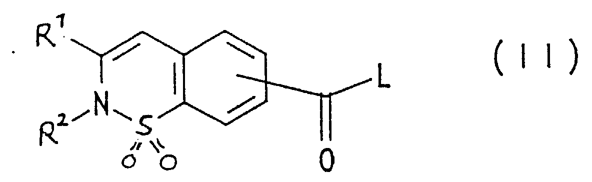 dans laquelle
R¹ et R² ont les significations données dans la revendication 1 et
L représente le groupe méthoxy, phénoxy, phénylthio, méthylthio, 2-pyridylthio, 1-imidazolyle, -OCOO-éthyle, -O-SO₂-p-tolyle ou -O-C (=N-cyclohexyl)-(NH-cyclohexyle).

5. Utilisation d'un composé I selon la revendication 1, pour la fabrication d'un médicament destiné au traitement d' arythmies.

6. Utilisation d'un composé I selon la revendication 1, pour la fabrication d'un médicament destiné au traitement ou à la prophylaxie de l'infarctus du myocarde.

7. Utilisation d'un composé I selon la revendication 1, pour la fabrication d'un médicament destiné au traitement ou à la prophylaxie de l'angine de poitrine.

8. Utilisation d'un composé I selon la revendication 1, pour la fabrication d'un médicament destiné au traitement ou à la prophylaxie d'états ischémiques du coeur.

9. Utilisation d'un composé I selon la revendication 1, pour la fabrication d'un médicament destiné au traitement ou à la prophylaxie d'états ischémiques du système nerveux périphérique et central et de l'apoplexie cérébrale.

10. Utilisation d'un composé I selon la revendication 1, pour la fabrication d'un médicament destiné au traitement ou à la prophylaxie d'états ischémiques d'organes périphériques et des membres.

11. Utilisation d'un composé I selon la revendication 1, pour la fabrication d'un médicament destiné au traitement d'états de choc.

12. Utilisation d'un composé I selon la revendication 1, pour la fabrication d'un médicament destiné à l'utilisation dans des opérations chirurgicales et des greffes d' organes.

13. Utilisation d'un composé I selon la revendication 1, pour la fabrication d'un médicament destiné à la conservation et au stockage de transplants pour des opérations chirurgicales.

14. Utilisation d'un composé I selon la revendication 1, pour la fabrication d'un médicament destiné au traitement de maladies dans lesquelles la prolifération cellulaire représente une cause primaire ou secondaire.

15. Médicament, contenant une quantité efficace d'un composé I selon la revendication 1, et des additifs usuels.
